(19) Europäisches Patentamt
European Patent Office
Office européen des brevets

(11) Publication number: **0 489 404 A2**

(12) **EUROPEAN PATENT APPLICATION**

(21) Application number: **91120768.6**

(22) Date of filing: **03.12.91**

(51) Int. Cl.5: **A61K 31/00**, A61K 31/35, A61K 31/40, A61K 31/415

(30) Priority: **05.12.90 US 622679**

(43) Date of publication of application: **10.06.92 Bulletin 92/24**

(84) Designated Contracting States: **AT BE CH DE DK ES FR GB GR IT LI LU NL SE**

(71) Applicant: **E.R. SOUIBB & SONS, INC.**
**P.O.Box 4000**
**Princeton New Jersey 08543-4000(US)**

(72) Inventor: **Grover, Gary J.**
**101 Bowne Station Road**
**Stockton, NJ 08559(US)**

(74) Representative: **Josif, Albert et al**
**Baaderstrasse 3**
**W-8000 München 5(DE)**

(54) **Method of treating shock using a potassium channel activator.**

(57) A novel method for reducing or preventing impairment of cardiac and peripheral function resulting from shock is disclosed. The method comprises administering to a patient in need thereof a potassium channel activator.

EP 0 489 404 A2

Shock is the term used generally to describe the various manifestations of an inadequate volume or a maldistribution of circulating blood accompanied by a series of progressively increasing physiologic adjustments, adverse to the cardiovascular system, which can lead to eventual refractoriness to treatment. Shock may be caused by blood loss, heart failure, burns, or other forms of trauma. A crucial element which must be considered in the successful management of shock is that of cardiac impairment and subsequent peripheral ischemia.

In the shock syndrome a substantial decrease in blood volume causes decreased cardiac output and decreased blood pressure. Included in the various physiological responses triggered by the decreased cardiac output and decreased blood pressure are increased release of vasoconstrictors and catecholamines, decrease in visceral and coronary blood flow and increased heart rate. The increased catecholamine and vasoconstrictor release and decreased coronary blood flow are believed to cause hypoxic conditions which can eventually result in necrotic subendocardial lesions. The increased heart rate along with the increased levels of catecholamines can also produce zonal lesions. The decreased visceral blood flow can result in release of a myocardial depressant factor. The necrotic and zonal lesions and release of myocardial depressant factor, in turn, further compromise the heart, e.g., in the forms of arrhythmia, fibrillation, ischemia, etc., and this results in continuation of the aforementioned cycles which results in yet more pronounced lesions and depressant factor release, causing greater fibrillation, arrhythmia and ischemia, eventually resulting in cardiac failure and irreversible collapse of the cardiovascular system.

Some prevention of zonal lesions during shock can be provided by surgically-induced heart block, administration of anti-beta-adrenergic agents, e.g., pronetholol, or cardiac denervation plus adrenalectomy. Necrotic lesions have been reportedly prevented by these same methods and also by hyperbaric oxygen treatment to avoid hypoxia.

Additional studies have shown the calcium channel blocker, verapamil, to provide improved cardiac function during shock. Verapamil is a vasodilator and lowers the heart rate. The vasodilatory effects are believed to increase coronary blood flow resulting in enhanced supply of oxygen to the heart muscle. The decrease in heart rate is believed to lower oxygen demand. Therefore, prevention of necrotic myocardial lesions has been reported using verapamil during shock.

In addition to cardiac damage, peripheral damage occurs secondary to cardiac insufficiency due to maladistribution of blood. An ideal agent would protect both the heart and the periphery.

In accordance with the present invention a novel method for reducing or preventing the occurrence of cardiac and peripheral impairment during shock is disclosed. The method comprises the administration of an effective amount of a potassium channel activator to a mammalian specie in shock.

As is understood in the medical art and as described above, shock, as the term is used in describing the present invention, refers to the clinical manifestations of an inadequate volume of circulating blood accompanied by the series of progressively increasing physiologic responses precipitated thereby, which responses are adverse to the cardiovascular system, including the heart. ATP-sensitive potassium channel openers have been shown to protect ischemic hearts and it has now been found that these agents also protect hearts during shock. Further, since ATP-sensitive potassium channels also exist in skeletal muscle and kidneys, which are often damaged during shock, these areas are protected as well. In practicing the present method the potassium channel activator should be administered as soon as possible in the shock episode and continued throughout the period of shock and in the post-shock period for a time sufficient to ensure cardiac protection. Potassium channel activators have also been found to possess excellent antiischemic, antifibrillatory and antiarrhythmic activities which are believed to provide enhanced cardiac protection during and after the shock event.

In certain situations, such as in the treatment of patients in shock who are also affected with a previously-compromised heart/cardiovascular system, it may be desirable to provide protection to the heart against shock-induced myocardial lesions, fibrillation, arrhythmia and ischemia without lowering blood pressure and further compromising the heart/cardiovascular system. In a preferred embodiment of the present invention there is provided a method for treating shock providing the above-metioned effects using a selective potassium channel activator which has little or no vasodilatory activity and does not further reduce blood pressure.

In addition to cardiac protection, potassium channel openers can protect skeletal muscle, brain and kidneys as these organs also possess potassium channels.

Any potassium channel activator may be used in accordance with the present invention. Suitable potassium channel activators include those disclosed in U. S. Patent 4,057,636, especially the compound

A

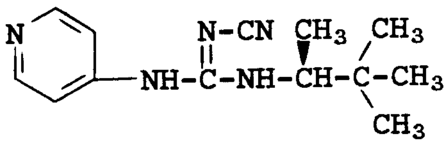,

known as pinacidil; those disclosed in European Patent Application 0 274 821, especially the compound

B

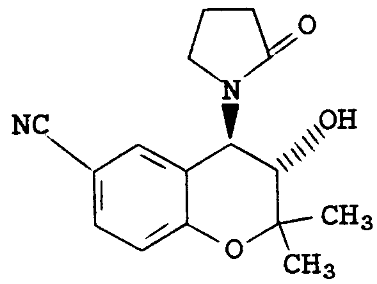

known as cromakalim; nicorandil; minoxidil; compounds in copending application U. S. Ser. No. 506,632 filed April 9, 1990 having the formula

C

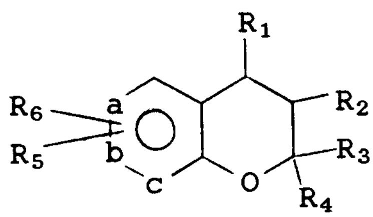

wherein a, b, and c are all carbons or one of a, b and c can be nitrogen or -NO- and the others are carbons;

$R_1$ is

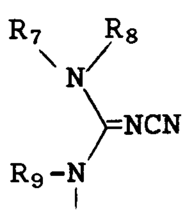 or 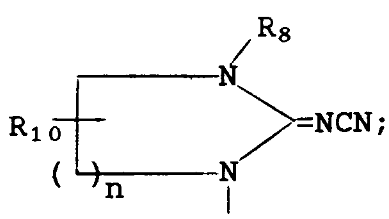

$R_2$ is hydrogen, hydroxy,

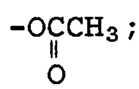

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

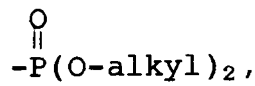, 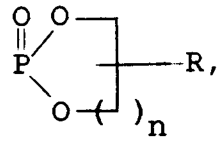

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, OH, O-alkyl, amino, substituted amino, CN, and $NO_2$;

$R_7$ and $R_8$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or $R_7$ and $R_8$ taken together with the nitrogen atom to which they are attached form 1-pyrrolidinyl, 1-piperidinyl, 1-azepinyl, 4-morpholinyl, 4-thiamorphilinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;

$R_9$ and $R_{10}$ are selected from hydrogen, alkyl, alkenyl, aryl, arylalkyl, cycloalkyl or cycloalkylalkyl;

n is 1, 2 or 3; and,

wherein the term "aryl" refers to phenyl, 1-naphthyl, 2-naphthyl or monosubstituted phenyl, 1-naphthyl, 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, $-N(alkyl)_2$ wherein alkyl is of 1 to 4 carbons, $-CF_3$, $OCHF_2$,

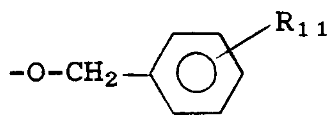,

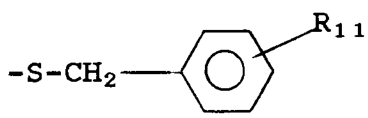

(wherein $R_{11}$ is hydrogen, alkyl of 1 to 4 carbons, alkoxy of 1 to 4 carbons, alkylthio of 1 to 4 carbons, halo, hydroxy or $CF_3$), $-O-CH_2$-cycloalkyl or $-S-CH_2$-cycloalkyl, and di-substituted phenyl, 1-naphthyl, 2-naphthyl wherein said substituents are selected from methyl, methoxy, methylthio, halo, $CF_3$, nitro, amino and $OCHF_2$;

with the compound

<u>C'</u>

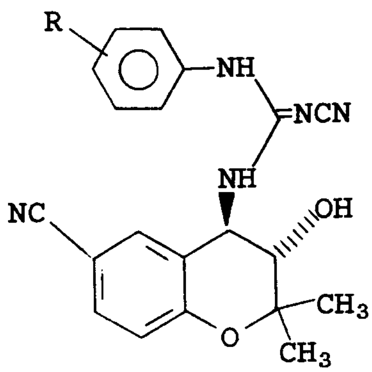

(where R can be alkyl of 1 to 4 carbons, halo, nitro, cyano, hydroxy, amino, -NH-alkyl of 1 to 4 carbons or $-N(alkyl)_2$ where alkyl is of 1 to 4 carbons)

being preferred; compounds in copending application U. S. Ser. No. 349,021 filed May 8, 1989 having the

formula

D 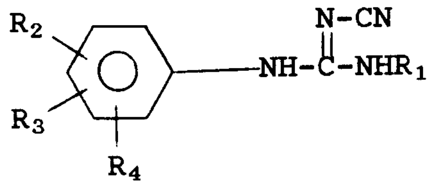

and its possible tautomers

D' 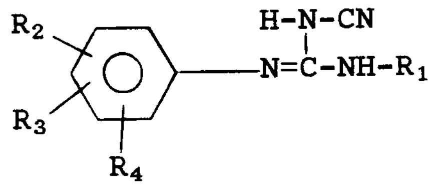

and

D'' 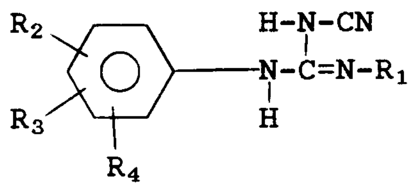

wherein $R_1$ is alkyl, alkenyl, alkynyl, haloalkyl, cycloalkyl, aryl, arylalkyl or cycloalkylalkyl;
$R_2$ is -C≡N, $-NO_2$,

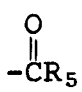 , 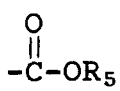 , 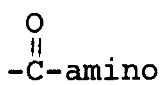 ,

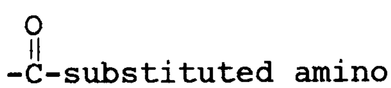 ,

$-CF_3$ or

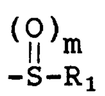 ;

$R_3$ and $R_4$ are each independently selected from $-R_2$, hydrogen, alkyl, alkenyl, alkynyl, haloalkyl, halo, alkoxy, -NHalkyl, $-N-(alkyl)_2$, -S-alkyl, -O-arylalkyl, -S-arylalkyl or -S-aryl, -O-aryl, -NHarylalkyl, or $R_2$ and $R_3$ taken together are a group which forms a ring with the two carbon atoms to which they are attached, which group is selected from

$$-\overset{(O)_m}{\overset{\|}{S}}-(CH_2)_n-CH_2-,$$

$$-\overset{O}{\overset{\|}{C}}X(CH_2)_pCH_2-,\quad -\overset{O}{\overset{\|}{C}}-CH_2(CH_2)_pX-;$$

wherein
m = 1 or 2,
n = 1-3,
p = 0-2,
X is 0, $NR_5$, $CH_2$; and
$R_5$ is hydrogen or $R_1$;

compounds disclosed in copending patent application serial number 540,423 filed June 18, 1990 of the formula

E

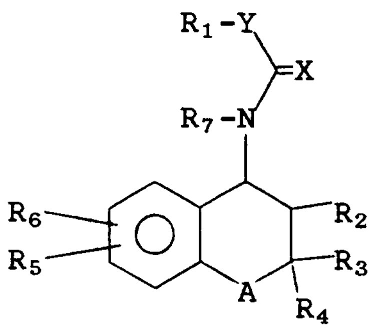

wherein A can be $-CH_2-$, -O-, $-NR_9$, -S-, -SO- or $-SO_2-$, where $R_9$ is hydrogen or lower alkyl of 1 to 4 carbons;

wherein X is oxygen or sulfur;

Y is $-NR_8$, -O-, -S- or

$$\overset{R_{10}}{\overset{|}{-CH-}};$$

$R_1$ is aryl, arylalkyl, heterocyclo or (heterocyclo)alkyl;

$R_2$ is hydrogen, hydroxy,

$$-O\underset{\underset{O}{\|}}{C}CH_3;$$

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

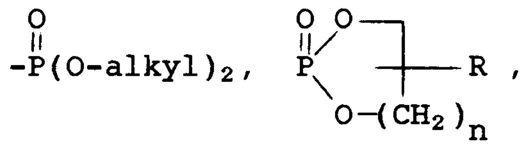

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, halo, OH, O-alkyl, amino and substituted amino;

$R_7$ and $R_8$ are each independently selected from hydrogen, alkyl, arylalkyl;

n is 1, 2 or 3; and,

$R_{10}$ is hydrogen, hydroxy, alkyl or O-alkyl; and compounds in copending application Serial No. 502,967 filed April 2, 1990 having the general formula

F

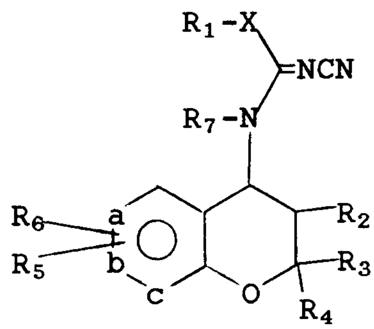

wherein a, b, and c are all carbons or one of a, b and c can be nitrogen or -NO- and the others are carbons; where X is oxygen or sulfur;

$R_1$ is selected from aryl, arylalkyl, (heterocyclo)alkyl, heterocyclo, cycloalkyl and (cycloalkyl)alkyl.

$R_2$ is hydrogen, hydroxy,

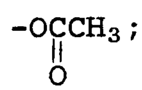

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

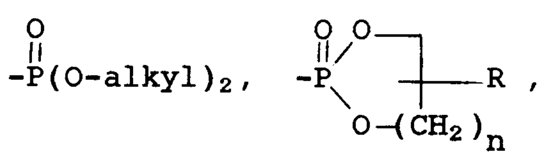

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, OH, O-alkyl, amino, substituted amino, CN, and $NO_2$;

$R_7$ is selected from hydrogen, alkyl, alkenyl, aryl, arylalkyl, cycloalkyl or cycloalkylalkyl; and,

n is 1, 2 or 3.

In carrying out the method of the present invention, the potassium channel activator may be administered to mammalian species, such as monkeys, dogs, cats, rats, humans, etc., during the period of shock.

The potassium channel activator may be administered systemically, such as orally or parenterally, or the potassium channel activator be administered locally to the coronary arteries by catheter such as by arterial angiography or intracoronary injection.

The potassium channel activator may be incorporated in a conventional dosage form, such as a tablet, capsule, elixir or injectable. The above dosage forms will also include the necessary carrier material, excipient, lubricant, buffer, antibacterial, bulking agent (such as mannitol), anti-oxidants (ascrobic acid of sodium bisulfite) or the like. Oral dosage forms are preferred, although parenteral forms are quite satisfactory as well.

With regard to such systemic formulations, single or divided doses of from about 5 to about 2500 mg, preferably from about 10 to 2000 mg/one to four times daily, may be administered in systemic dosage forms as described above for a period sufficient to reduce and avoid impairment of cardiac function resulting from the shock event.

With regard to dosage of potassium channel activator, where the drug is administered by arterial angiography or intracoronary injection, from about 0.001 to about 30 mg/kg/treatment and preferably from about 0.5 to about 25 mg/kg/treatment will be employed. The number of treatments will depend upon the severity of the fibrillation and the progress of reperfusion to achieve normal heart rhythm. Usually, from 1 to 5 treatments per day will be required for as long as fibrillation continues.

Where the potassium channel activator is to be administered by angiography or intracoronary injection, it will be formulated in a conventional vehicle, such as distilled water, saline, Ringer's solution, or other conventional carriers.

The preferred method of the present invention involves using compounds of the general formulae C, E or F to maintain cardiac function during and after shock without lowering blood pressure. Compounds of formula C, E or F are useful in the parent method where no antihypertensive action is desired. Such "selective" compounds are those potassium channel activators which have $IC_{50}$ (rat aorta) values greater than that of cromakalim. These include compounds of formula C where $R_7$ is (or compounds of formula E or F where $R_1$ is) aryl, arylalkyl, heteroaryl or heteroarylalkyl.

Grover et al., "Dissociation of Cardiodepression from Cardioprotection with Calcium Antagonists: Diltiazem Protects Ischemic Rat Myocardium with a Lower Functional Cost as Compared with Verapamil and Nifedipine", Journal of Cardiovascular Pharmacology; pages 331-340, Vol. 14, No. 2 (1989), describe a model for testing globally ischemic, isolated rat hearts. This model is expected to be a reliable indicator of protection since the laboratory-induced isolation and ischemic event including perfusion with a cardioplegic solution, reasonably duplicates the environment and conditions for the heart during and after a shock or trauma episode. Grover et al. express the efficacy of protective agents as the amount of lactate dehydrogenase (LDH) release and post-ischemic cardiac function.
Lactate dehydrogenase is an enzyme released in the heart during an ischemic event and is an index of cardiac cell necrosis. In the Grover et al. model, this is measured during reperfusion and an agent which provides for lower release levels of LDH is considered to offer greater cardioprotection since lower LDH indicates a smaller infarct size. Cardiac function is determined using the double product (DP) of heart rate times the left ventricular developed pressure (LVDP) divided by 1,000.

The lower the value for DP before the ischemic isolation of the heart for a given agent, the more cardiodepressant it is considered to be and the higher the value of DP is during reperfusion, the more cardioprotective the agent is.

The following Example examines cromakalim, a potassium channel activator having vasodilator/blood pressure lowering activity, and compounds from formula C, E and F, which have little or no vasodilating activity in normal tissue.

Compounds Tested in Example

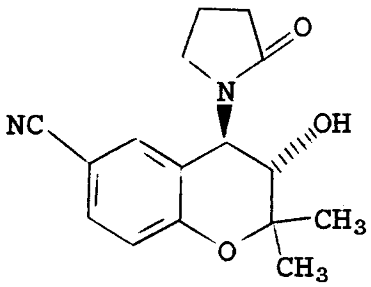

Cromakalim

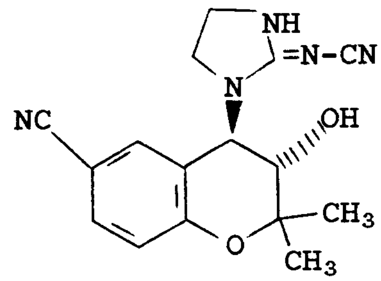

C''

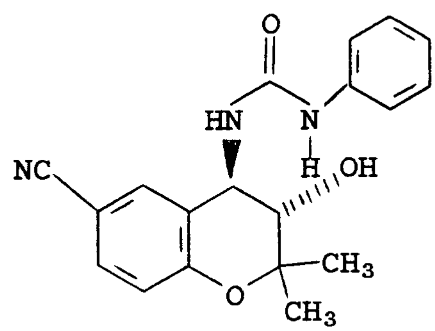

E''

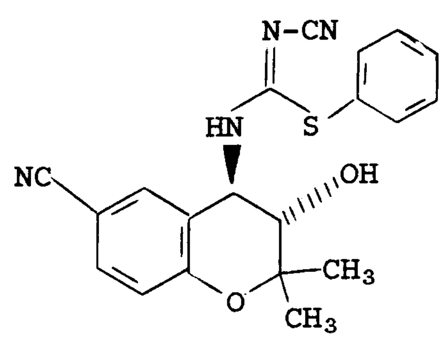

F''

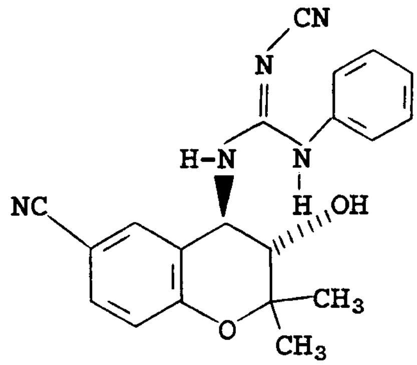

C'''

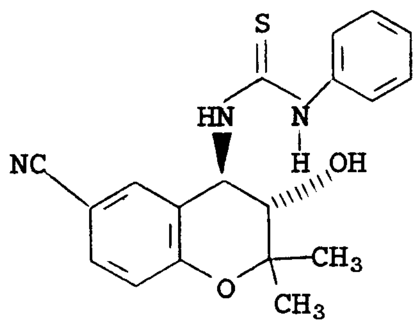

E'

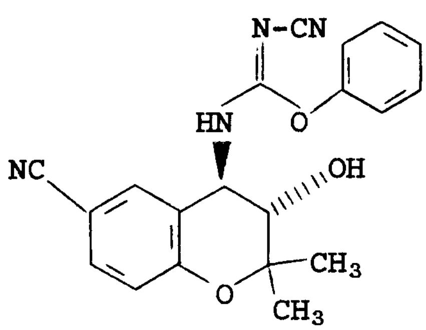

F'

EXAMPLE

Preparation of the Isolated Perfused Hearts

Male Sprague-Dawley rats (450-550 g) were used in all experiments. The rats were anesthetized using 30 mg/kg sodium pentobarbital (i.p.). They were intubated and then treated with i.v. heparin (1000 U/kg). While being mechanically ventilated, their hearts were perfused *in situ* via retrograde cannulation of the aorta. The hearts were then excised and quickly moved to a Langendorff apparatus where they were perfused with Krebs-Henseleit bicarbonate buffer (112 mM $NaCl_2$, 25 mM $NaHCO_3$, 5 mM KCl, 1.2 mM $MgSO_4$, 1 mM $KH_2PO_4$, 1.25 mM $CaCl_2$, 11.5 mM dextrose, and 2 mM pyruvate bubbled with 95% $O_2$ - 5% $CO_2$) at a constant pressure (75 mm Hg). A water filled latex balloon attached to a metal cannula was then inserted into the left ventricle and connected to a Statham pressure transducer for measurement of left ventricular pressure. The hearts were allowed to equilibrate for 15 minutes at which time end diastolic pressure (EDP) was adjusted to 5 mm Hg and this was maintained for 5 minutes. Pre-ischemia or pre-drug function, heart rate and coronary flow (extracorporeal electromagnetic flow probe, Carolina Medical Electronics, King, N.C.) were then measured. Cardiac function was determined using the double product of heart rate (HR) X left ventricular developed pressure (LVDP) divided by 1000. Cardiac temperature was maintained throughout the experiment by submerging the hearts in 37°C buffer which was allowed to accumulate in a stoppered, heated chamber.

Experimental Protocol

Once the baseline measurements were taken, the hearts were treated with 10 µM cromakalim, compounds C', C'', E', E'', F', F'' (n = 4 each) or with vehicle buffer (0.01% DMSO, n = 7). All of these hearts were treated with their respective drugs or vehicle for ten minutes. At this time, post-drug cardiac function and flow were measured and then the hearts were made globally ischemic by shutting off the buffer perfusion. The ischemia was maintained for 25 minutes, the hearts were then reperfused with nondrug treated buffer. Reperfusion was maintained for a total of 30 minutes and at this time reperfusion function and flow were again determined. The results are summarized in the TABLE below.

Also included in the TABLE are the $IC_{50}$(m) values for rat aorta. The $IC_{50}$ (rat aorta) value is the concentration of the particular compound which inhibits agonist-induced constriction in rat aorta by 50 percent. Thus, the lower values indicate greater vasodilation and it should be noted that these values are for normal, i.e., non-ischemic, tissue. It can be seen that cromakalim with an $IC_{50}$ of $5.7 \times 10^{-8}$ m is a relatively potent vasodilator in non-ischemic tissue. The ischemia selective compounds, however, are comparable in the anti-ischemic effects (LDH) but have only a fraction of the vasodilator action in non-ischemic tissue.

TABLE

Protective Effect of Potassium Channel Activators

Ischemic Isolated Rat Heart

| Compound | Pre-ischemic Event (Post Drug) HR | LVDP | DP | FLOW | Reperfusion (Post-Ischemic Event) HR | LVDP | DP | FLOW | LDH | $IC_{50}$(m) Rat Aorta |
|---|---|---|---|---|---|---|---|---|---|---|
| Vehicle | 245 | 144 | 35.5 | 15.5 | 186 | 28 | 5.6 | 14.7 | 21.94 | --- |
| Cromakalim | 250 | 131 | 33.0. | 23.9 | 234 | 87 | 20.5 | 13.9 | 8.89 | $5.7 \times 10^{-8}$ |
| C'" | 244 | 134 | 32.7 | 15.2 | 244 | 74 | 18.2 | 13.2 | 10.41 | $1.37 \times 10^{-6}$ |
| C" | 250 | 121 | 29.8 | 20.4 | 240 | 62 | 14.8 | 10.1 | 9.88 | $4.47 \times 10^{-7}$ |
| E' | 236 | 141 | 32.75 | 15.3 | 229 | 55 | 14.83 | 13.4 | 13.46 | $1.71 \times 10^{-6}$ |
| E" | 234 | 128 | 30.06 | 24.8 | 230 | 81 | 18.52 | 17.56 | 8.77 | $8.05 \times 10^{-7}$ |
| F' | 238 | 128 | 30.5 | 16.0 | 212 | 28 | 6.2 | 10.4 | 21.7 | $>1 \times 10^{-4}$ |
| F" | 239 | 159 | 38.02 | 28.3 | 247 | 59 | 14.72 | 13.92 | 10.65 | $4.9 \times 10^{-6}$ |

Concentration: 10 μM

Occlusion: 25 minutes

Reperfusion: 30 minutes

HR = Heart Rate

LVDP = Left Ventricular Developed Pressure (mmHg)

DP = Double Product

LDH = Lactate Dehydrogenase Release

## Claims

1. Use of a potassium channel activator for preparing a medicament for reducing or preventing the impairment of cardiac function caused by shock in a mammalian specie in need thereof.

**2.** The use of claim 1 wherein said potassium channel activator is for administration as soon as possible after initiation of the shock occurrence with said administration to be continued for a time sufficient to ensure cardiac and peripheral protection.

**3.** The use of claim 1 wherein said potassium channel activator is selected from nicorandil, minoxidil, pinacidil, cromakalim and compounds of the formula

C

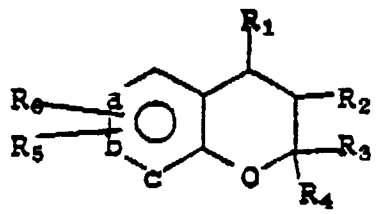

wherein a, b, and c are all carbons or one of a, b and c can be nitrogen or -NO- and the others are carbons;

$R_1$ is

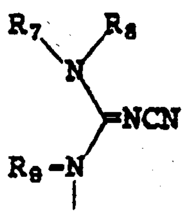 or 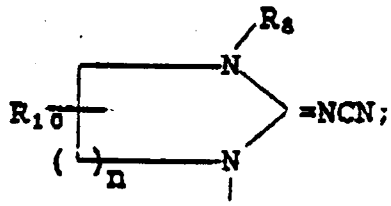

$R_2$ is hydrogen, hydroxy,

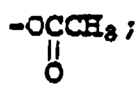

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

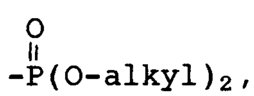

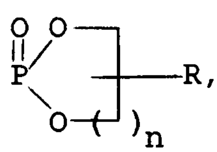

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, OH, O-alkyl, amino, substituted amino, CN, and $NO_2$;

$R_7$ and $R_8$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or $R_7$ and $R_8$ taken together with the nitrogen atom to which they are attached form 1-pyrrolidinyl, 1-piperidinyl, 1-azepinyl, 4-morpholinyl, 4-thiamorphilinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;

$R_9$ and $R_{10}$ are selected from hydrogen, alkyl, alkenyl, aryl, arylalkyl, cycloalkyl or cycloalkylalkyl;
n is 1, 2 or 3; and,

wherein the term "aryl" refers to phenyl, 1-naphthyl, 2-naphthyl or monosubstituted phenyl, 1-naphthyl, 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)$_2$ wherein alkyl is of 1 to 4 carbons, $-CF_3$, $OCHF_2$,

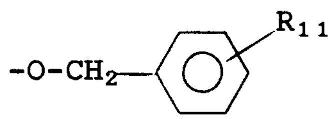
,

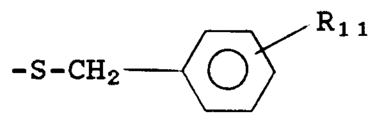

(wherein $R_{11}$ is hydrogen, alkyl of 1 to 4 carbons, alkoxy of 1 to 4 carbons, alkylthio of 1 to 4 carbons, halo, hydroxy or $CF_3$), $-O-CH_2$-cycloalkyl or $-S-CH_2$-cycloalkyl, and di-substituted phenyl, 1-naphthyl, 2-naphthyl wherein said substituents are selected from methyl, methoxy, methylthio, halo, $CF_3$, nitro, amino and $OCHF_2$;

D

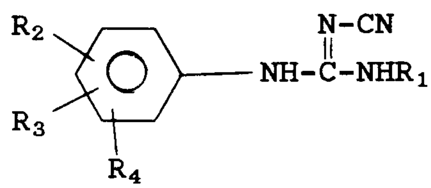

wherein $R_1$ is alkyl, alkenyl, alkynyl, haloalkyl, cycloalkyl, aryl, arylalkyl or cycloalkylalkyl;
$R_2$ is -C≡N, $-NO_2$,

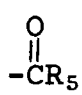, 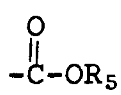, 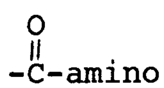,

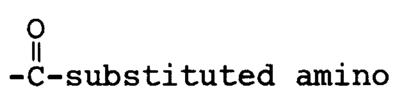,

$-CF_3$ or

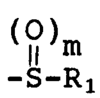;

$R_3$ and $R_4$ are each independently selected from $-R_2$, hydrogen, alkyl, alkenyl, alkynyl, haloalkyl, halo, alkoxy, -NHalkyl, -N-(alkyl)$_2$, -S-alkyl, -O-arylalkyl, -S-arylalkyl or -S-aryl, -O-aryl, -NHarylalkyl, or $R_2$ and $R_3$ taken together are a group which forms a ring with the two carbon atoms to which they are attached, which group is selected from

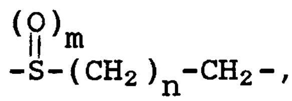

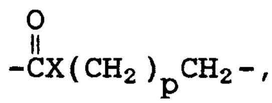

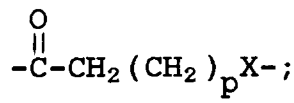

wherein
m = 1 or 2,
n = 1-3,
p = 0-2,
X is 0, $NR_5$, $CH_2$; and
$R_5$ is hydrogen or $R_1$;

E

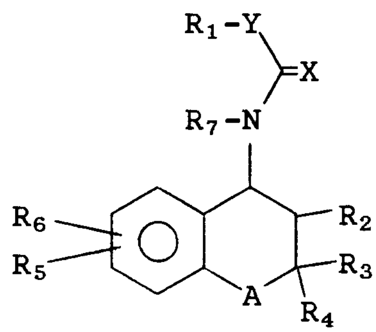

wherein A can be $-CH_2-$, -O-, $-NR_9-$, -S-, -SO- or $-SO_2-$, where $R_9$ is hydrogen or lower alkyl of 1 to 4 carbons;

wherein X is oxygen or sulfur;

Y is $-NR_8$, -O-, -S- or

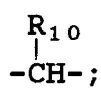

$R_1$ is aryl, arylalkyl, heterocyclo or (heterocyclo)alkyl;

$R_2$ is hydrogen, hydroxy,

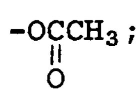

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

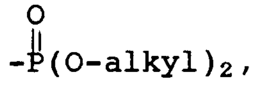

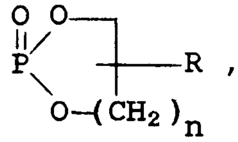

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, halo, OH, O-alkyl, amino and substituted amino;

$R_7$ and $R_8$ are each independently selected from hydrogen, alkyl, arylalkyl;

n is 1, 2 or 3; and,

$R_{10}$ is hydrogen, hydroxy, alkyl or O-alkyl; and

F

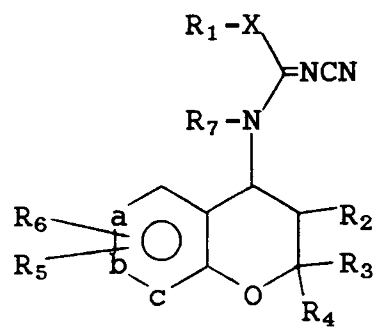

wherein a, b, and c are all carbons or one of a, b and c can be nitrogen or -NO- and the others are carbons;

where X is oxygen or sulfur;

$R_1$ is selected from aryl, arylalkyl, (heterocyclo)alkyl, heterocyclo, cycloalkyl and (cycloalkyl)alkyl.

$R_2$ is hydrogen, hydroxy,

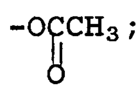

$R_3$ and $R_4$ are each independently hydrogen, alkyl or arylalkyl, or, $R_3$ and $R_4$ taken together with the carbon atom to which they are attached form a 5- to 7-membered carbocyclic ring;

$R_5$ is selected from H, alkyl, haloalkyl, alkenyl, alkynyl, cycloalkyl, arylalkyl, cycloalkylalkyl, -CN, $-NO_2$, -COR, -COOR, -CONHR, $-CONR_2$, $-CF_3$, S-alkyl, -SOalkyl, $-SO_2$alkyl,

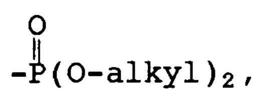, 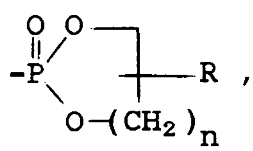,

halogen, amino, substituted amino, O-alkyl, $OCF_3$, $OCH_2CF_3$, -OCOalkyl, -OCONRalkyl, -NRCOalkyl and NRCOOalkyl, $NRCONR_2$ wherein R in each of the above groups can be hydrogen, alkyl, aryl, arylalkyl, cycloalkyl, or (cycloalkyl)alkyl;

$R_6$ is selected from H, alkyl, OH, O-alkyl, amino, substituted amino, CN, and $NO_2$;

$R_7$ is selected from hydrogen, alkyl, alkenyl, aryl, arylalkyl, cycloalkyl or cycloalkylalkyl; and,

n is 1, 2 or 3.

**4.** The use of claim 1 wherein said potassium channel activator has an $IC_{50}$ (rat aorta) value greater than said value for the potassium channel activator, cromakalim.

**5.** The use of claim 4 wherein said potassium channel activator is selected from

C

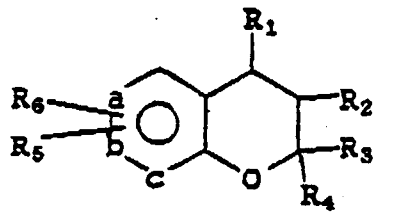

D

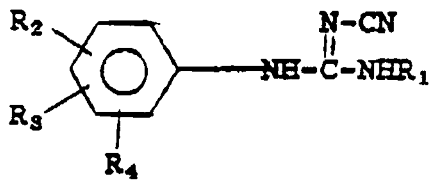

E

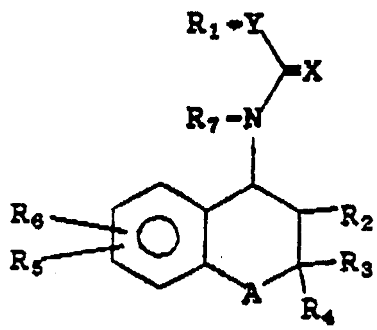

or

F

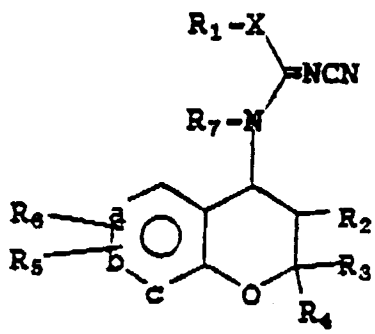

**6.** The use of claim 5 wherein said potassium channel activator is

C'"

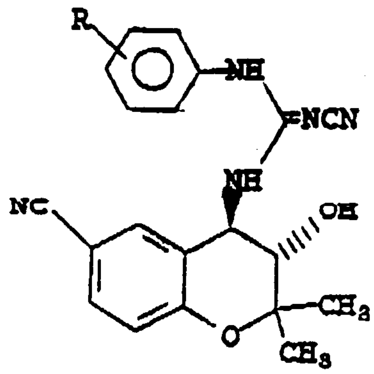

where R can be alkyl of 1 to 4 carbons, halo, nitro, cyano, hydroxy, amino, -NH-alkyl of 1 to 4 carbons or $-N(alkyl)_2$ where alkyl is of 1 to 4 carbons.